# EUROPEAN PATENT SPECIFICATION

(11) **EP 1 593 011 B1**
(45) Date of publication and mention of the grant of the patent: **20.08.2008**
(21) Application number: 04710924.4
(22) Date of filing: 13.02.2004
(51) Int. Cl.: G05D 1/02

(54) **AN AUTONOMOUS MACHINE**
AUTONOME MASCHINE
MACHINE AUTONOME

(30) Priority: 14.02.2003 GB 0303368
(43) Date of publication of application: 09.11.2005
(73) Proprietor: Dyson Technology Limited, Malmesbury, Wiltshire SN16 0RP (GB)
(72) Inventor: ALDRED, Michael David, Wiltshire SN14 6RS (GB); BOMMER, Alexander Philip, Wiltshire BS3 1RZ (GB)
(74) Representative: Hucker, Nerys
(86) International application number: PCT/GB2004/000587
(87) International publication number: WO 2004/072750

(56) References cited:
- EP-A- 1 003 088
- DE-A- 10 064 836
- GB-A- 1 252 134
- US-A- 5 537 017
- US-A- 5 867 800

## Description

This invention relates to an autonomous machine for performing a task which requires the machine to traverse an area. The invention is particularly suitable for, but not limited to, an autonomous surface-treating machine such as an autonomous vacuum cleaner, for cleaning a room of a building.

There have been various proposals to provide autonomous or robotic machines for performing duties such as cleaning or polishing a floor area, or for mowing grass. A typical autonomous machine has a control system in which is stored a map of the working area. The machine uses this map to plan a route around the working area and/or to travel around the area in accordance with a predetermined sequence.

There have also been proposals for autonomous machines that are capable of exploring the environment in which they are placed without advance knowledge of the layout of the environment. The machine explores the environment during a learning phase and subsequently uses this information during a working phase. The information may take the form of a map of the working area. Alternatively, the learning phase may be omitted, the machine simply constructing a map of the working area as it works.
EP1003088 is an example of an autonomous machine of the prior art.

A problem which may be encountered with such autonomous machines is that the control system needs to determine when the machine has completed a trip around the entire perimeter of the area and returned to a starting point. Odometry errors, for example, can cause the machine to fail to recognise when it has reached this point.

The invention provides an autonomous machine comprising a main body, a power cable for providing power to the main body, a marker and a control system arranged to cause the main body to traverse a working area according to a predetermined sequence, the control system including a detector arranged to detect the marker, one of the detector and the marker being located on the main body, the other of the detector and the marker being locatable at a plurality of positions on the cable, and the control system being arranged to recognise a predetermined location in the area, or to effect a predetermined part of the sequence, on detecting the marker.

The provision of a marker associated with the control system permits the machine to establish a reference point, in order to initiate predetermined parts of the sequence. Thus, the machine can acknowledge a start point for the sequence on detecting the marker. The machine can alternatively, or additionally, detect when it has completed a circuit of the area. Furthermore, the machine can detect when it has returned to the start point, and thus initiate the final portion of the sequence.

Preferably, the user positions the marker at a convenient location on the cable by sliding the marker along the cable.

Advantageously, the marker is magnetic, in which case a suitable detector would be a Hall effect sensor or a reed switch.

The autonomous machine can take many forms: it can be a floor treating machine such as a vacuum cleaner or floor polisher, a lawn mower or a robotic machine which performs some other function. Alternatively, it could be a general purpose robotic vehicle which is capable of carrying or towing a work implement chosen by a user.

The invention will now be described, by way of example, with reference to the accompanying drawings, in which:-
Figure 1 shows an autonomous machine constructed according to the invention in the form of a vacuum cleaner;
Figure 2 is a schematic side view of the cleaner of Figure 1;
Figures 3 and 4 are plan views showing the machine at work in a working area;
Figure 5 is a plan view showing the machine in another working area; and
Figures 6 and 7 are plan views illustrating ways in which the machine copes with large working areas.

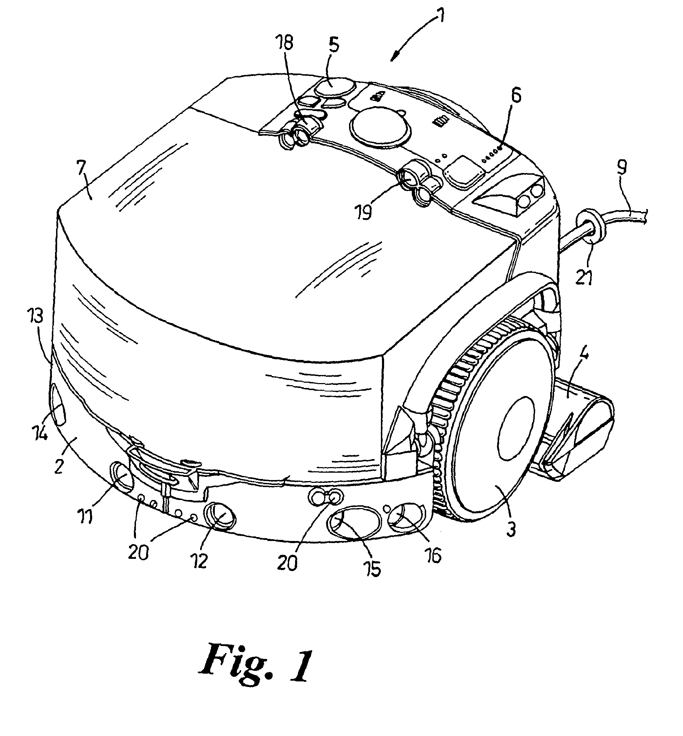

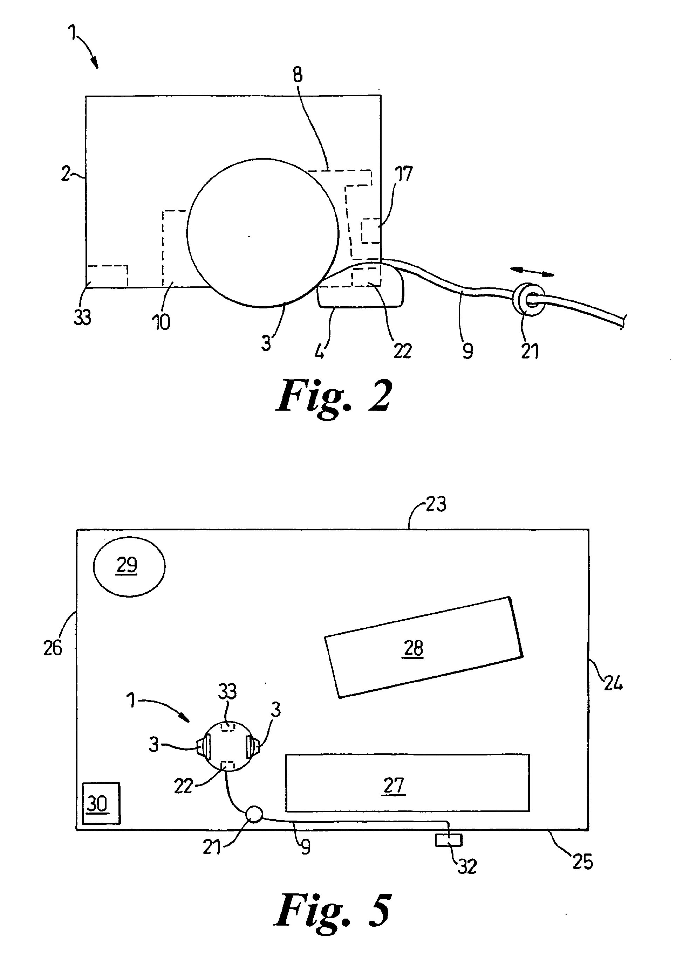

Figures 1 and 2 of the drawings shows a robotic, or autonomous, floor cleaning machine in the form of a robotic vacuum cleaner indicated generally by the reference numeral 1. The machine comprises a main body or supporting chassis 2, two driven wheels 3 and a cleaner head 4. A user interface with buttons 5 and indicator lamps 6 is provided. Also mounted on the chassis 2 is apparatus 7 for separating dirt, dust and debris from an incoming airflow and for collecting the separated material, an internal reel 8 for storing a length of power cable 9, and a system for dispensing and rewinding the power cable.

The machine 1 is supported on the two driven wheels 3 and a castor wheel (not shown) at the rear of the machine. The driven wheels 3 are arranged at either end of a diameter of the chassis 2, the diameter lying perpendicular to the longitudinal axis of the cleaner 1. The driven wheels 3 are mounted independently of one another via support bearings (not shown). Each driven wheel 3 is connected directly to a traction motor 10 which is capable of driving the respective wheel 3 in either a forward direction or a reverse direction. A full range of manoeuvres are possible by independently controlling each of the traction motors 10.

Mounted on the underside of the chassis 2 is a cleaner head 4 which includes a suction opening facing the surface on which the chassis is supported. A brush bar (not shown) may be rotatably mounted in the suction opening, in which case a motor may be mounted on the cleaner head 4 for driving the brush bar. For other types of surface treating machine, the cleaner head 4 could be replaced by a polishing pad, wax dispenser, squeegee etc.

The chassis 2 of the machine 1 carries a plurality of sensors 11 to 20, which are positioned on the chassis 2 such that the navigation system of the machine can sense obstacles in the path of the machine 1 and also the proximity of the machine to a wall or other boundary such as a piece of furniture. The sensors shown here comprise several ultrasonic sensors 11 -19, which are capable of sensing the distance and angular position of walls and objects from the sensors, and several passive infra red (PIR) sensors 20 which can sense the presence of humans, animals and heat sources such as a fire. There are forward-facing sensors 11, 12, side-facing sensors 13, 14, and 15, 16, rear-facing sensors 17 (Figure 2) and high-level sensors 18, 19. It will be appreciated that the number of sensors, type of sensors and positioning of the sensors on the machine 1 can take many different forms. For example, infra red range-finding devices, also known as PSDs, may be used instead of, or in addition to, the ultrasonic sensors. In an alternative embodiment the machine may navigate by mechanically sensing the boundary of the working area and boundaries of obstacles placed within the area. One example of a mechanical sensor which could be used on a machine of this type is a "bump" sensor which detects movement of a moveable or resilient bumper when the machine encounters an obstacle. "Bump" sensors can be used in combination with the ultrasonic and PIR sensors described above.

One or both sides of the vehicle can also have an odometry wheel (not shown). This is a non-driven wheel which rotates as the machine moves along a surface. Each odometry wheel has an encoder associated with it for monitoring the rotation of the odometry wheel. By examining the information received from each odometry wheel, the navigation system can determine both the distance travelled by the machine and any change in angular direction of the machine. It is preferred that the odometry wheel is a non-driven wheel as this increases the accuracy of the information obtained from the wheel. However, in a simpler embodiment, the machine can derive odometry information directly from the driven wheels 3, by an encoder located on the wheel or the motor 10 which drives the wheel 3.

The machine 1 also includes a motor and fan unit supported on the chassis 2 for drawing dirty air into the machine via the suction opening in the cleaner head 4.

The control system receives inputs about the environment surrounding the machine from the sensor array (including ultrasonic, PIR and bump sensors) and inputs about movement of the machine from odometry wheel movement sensors. The navigation system also receives inputs from switches 5 on the user interface, such as starting, pause, stop or a selection of operating speed or standard of required cleanliness. The navigation system provides a plurality of output control signals including signals for driving the traction motors 10 of the wheels 3, a signal for operating the suction motor which drives the suction fan and a signal for operating the motor which drives the brush bar. It also provides outputs from illuminating indicator lamps 6 on the user interface. Power is derived from a mains supply via a power cable. The cleaner carries a cable reel 8 with a length of cable 9 (e.g. 20m) which is sufficient to allow the machine to circumnavigate a typical room in which the machine will be used.

Figure 2 is a simplified diagram of the autonomous machine of the invention, showing the machine 1 from one side. In accordance with the invention, the machine 1 includes a marker 21 and a detector 22 capable of detecting the marker. In this embodiment, the detector 22 is located on the chassis 2 and the marker 21 is spaced from that main body by being located on the power cable 9 for the machine.

The marker 21 comprises an annulus of magnetic material, which is slidably positionable on the cable 9. Accordingly, the detector 22 comprises a magnetic field sensor, such as a Hall effect sensor or a reed switch. The detector 22 is located on the rear of the machine, preferably behind the wall comprising the rear portion of the chassis 2. When the rear portion of the chassis 2 of the machine is adjacent the marker 21, the detector 22 detects the marker and sends a signal to the control system.

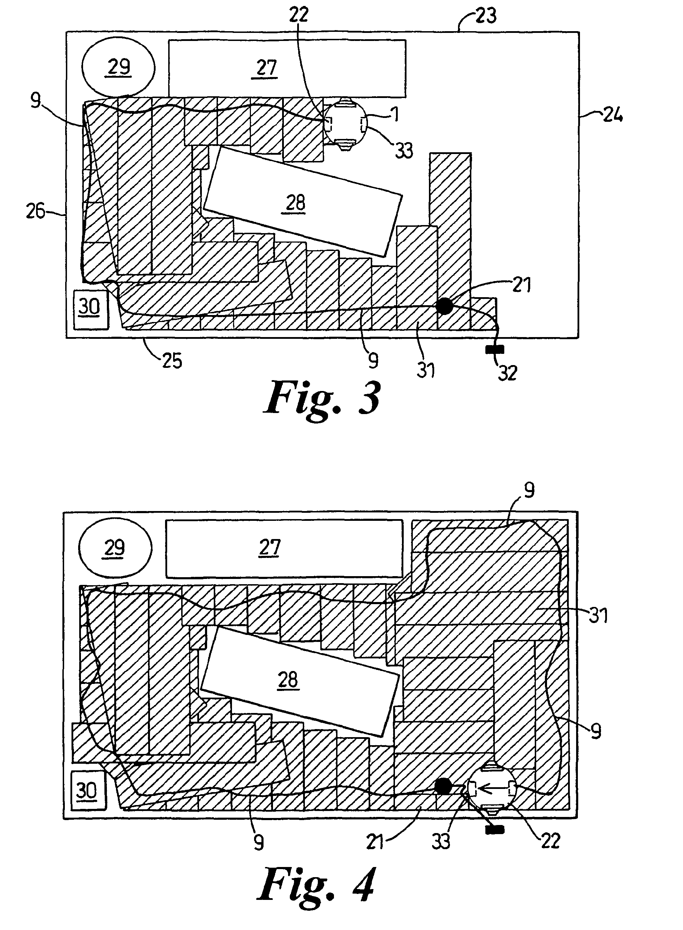

Figures 3 to 7 illustrate the autonomous machine in use in a room of a house. The boundary of the working area for the machine is defined by the walls of the room 23 - 26 and the edges of objects 27 - 30 placed within the room, such as articles of furniture (e.g. sofa, table, chair). These figures also show a typical set of paths 31 traversed by the machine.

A user places the machine 1 in the room. Ideally, the chassis 2 is positioned close to a power socket 32 in the room connected to the mains supply. The user then manually deploys a short length of power cable 9 from the internal cable reel 8 sufficient to allow the cable to be plugged into the socket 32. Of course, there may be situations in which it is simply not convenient or possible to place the chassis 2 of the machine close to a socket 32. For example, as is shown in Figure 5, an obstacle such as a sofa 27 may be positioned in front of the socket 32, so that the machine has to be placed on the floor some distance from the socket. In this situation, the user unwinds a longer length of power cable 9 so as to connect the machine to the mains supply. The user also positions the marker 21 at a convenient point such that the marker is located in the working area of the room. The marker 21 is preferably slidably moveable along the cable 9.

Once the machine has been switched on, it begins a short routine to discover a starting or 'home' position in the room. The 'home' position serves as a useful reference point for determining, inter alia, when the machine has travelled around the entire room. The machine winds the cable 9 back onto its internal cable reel 8 as it reverses. When the detector 22 detects the marker, the control system establishes this as the reference point and initiates the start of the sequence by which the chassis 2 traverses and treats the surface of the working area. The machine starts by aligning the left-hand side of the chassis with the boundary of the area and starts the suction motor and brush bar motor. It waits until the motors reach operating speed and then moves off. As the cleaner moves forward, it dispenses power cable 9 from the cable reel 8 so that the cable lies substantially along the path taken by the machine.

The machine then begins a series of manoeuvres in accordance with the predetermined sequence. The series of manoeuvres may comprise, for example, random movements, a spiral pattern, a so-called 'spike' pattern, or any combination of movement types. In a typical spike pattern, the machine turns so that it is pointing away from the boundary (wall), inwards into the working area. It travels forwards on a path which is substantially perpendicular to the boundary. The machine derives information on the distance and direction of travel from the odometry wheel sensors. As the cleaner moves forwards, it dispenses sufficient power cable 9 from the cable reel 8 so that the cable lies slackly along the path. During this movement, the machine continually monitors inputs from the sensor array 11-20, to sense the presence of any obstacles in its path. The machine continues to travel forwards until one of a number of conditions is met. Should the machine sense the presence of an obstacle or the absence of a surface (e.g. a staircase), or if the machine senses that it has dispensed all of the power cable 9 from the reel 8, or if it senses some other fault condition, it will immediately stop. If none of these conditions are met, the machine will stop after a predetermined distance has been travelled from the boundary. This distance will depend on the type of working area where the vehicle is working. In a domestic environment we have found that a maximum distance of 2-3m works well.

Once the machine has stopped, having met one or more of the conditions mentioned above, it reverses back towards the boundary following a similar path. The machine rewinds cable 9 during this return manoeuvre. For best cleaning performance, the suction motor and brush bar motor are operated during this return manoeuvre so as to treat the same area of floor twice. This replicates the 'to and fro' cleaning action that a human user performs when they use a vacuum cleaner. As an alternative, during this return manoeuvre the suction motor and brush bar motor can be switched off. This would be a useful way of increasing battery life for a battery powered machine. During the return manoeuvre, the machine can navigate towards the boundary by using odometry information or it can follow the cable 9 which was laid on the floor during the outward trip. This outward trip into the working area and back again to the boundary constitutes the previously mentioned 'spike'.

Once the machine has returned to the boundary, which it can sense from its sensor array and odometry information, it turns so that it is once again pointing in a clockwise direction, with its left-hand side aligned with the boundary. It moves forwards for a short distance which is sufficient to bring the machine next to the strip of the floor which has just been treated. The cleaner then turns so that it is again pointing away from the boundary, inwards into the working area. The machine then travels forwards at an angle which is substantially perpendicular to the boundary, as before. The machine continues as previously described, traversing a strip of the floor surface which is adjacent, or overlaps, the area previously treated.

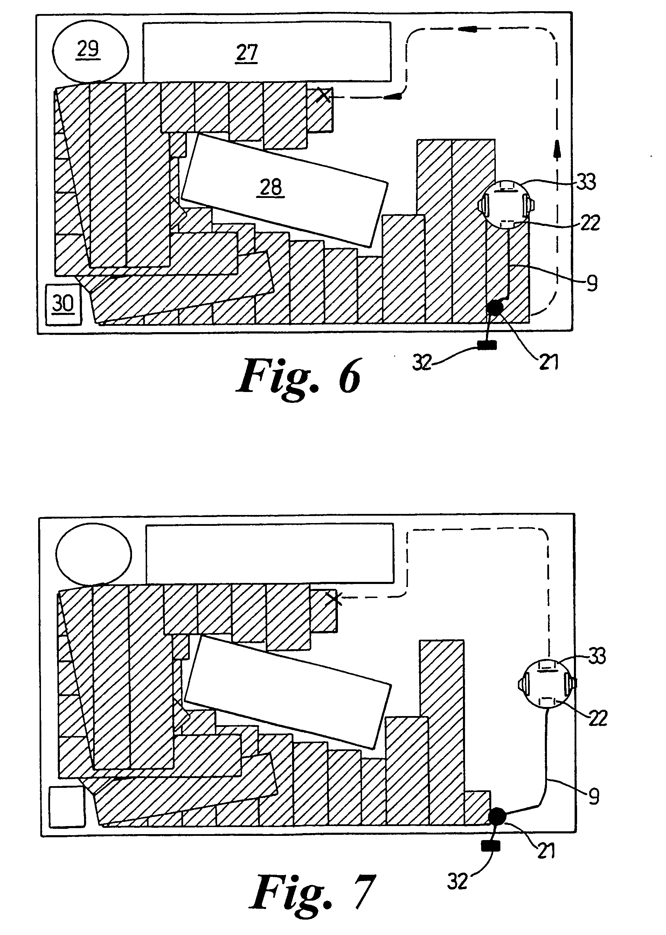

The machine repeats this sequence of steps so as to traverse a plurality of paths extending into the working area from the boundary, as can be seen in Figures 5 and 6. As the machine progresses around the boundary it can be seen that the spikes originating at different parts of the boundary can overlap one another. This helps to ensure that as much of the working area as possible is treated by the machine.

The machine needs to recognise when it has completed a circuit of the room. In accordance with an exemplary embodiment of the invention, the machine is provided with a second detector 33 on the front of the chassis 2. Preferably, the detector 33 is located behind a wall that comprises the front portion of the chassis 2. As the chassis 2 approaches the marker 21, the second detector 33 detects the marker and sends a signal to the control system. The control system is arranged to recognise that a signal from the second detector 33 is indicative that the machine has completed a circuit. The control system is then arranged to cause the machine to perform the next part of the control sequence. This may comprise a further cleaning operation in the opposite direction, or else the machine may be arranged to simply reverse back to the starting position in the working area.

The machine can follow the boundary of the working area, rewinding the cable 9 as it moves around the boundary. Alternatively, the machine can operate in so-called cable follow mode, in which it rewinds the cable 9 and follows the path formed by the cable on the surface of the working area. In the event that this brings the machine near to an obstacle, the machine can revert to a boundary following mode of operation until it is determined that the cable 9 leads away from the obstacle, whereupon the machine can once again operate in cable follow mode.

When the machine has made its way back around the room, it recognises that it has reached the home position indicated by the marker 21 once more by means of the first detector 22 on the rear of the chassis 2. The control system recognises that a subsequent signal from the first detector 22 is indicative that the machine has finished its cleaning operation around the working area. The control system may be arranged to complete the sequence by, for example, turning off the suction motor or by causing the machine to travel to another working area. The control system may also be arranged to sound an alarm to the user and/or to cause the indicator lamps 6 to flash or change colour. Alternatively, the machine may be arranged to assume a standby mode, or else may switch itself off completely.

In large working areas the machine may run out of cable before it has completely covered the working area. In this case, the machine proceeds to perform the same technique as has previously been described in the opposite direction from the starting point. Thus, the cleaner follows the boundary in an anti-clockwise direction, aligning the right-hand side of the machine with the boundary of the area and performing a series of spikes outwardly from the boundary of the working area.

Figure 6 shows the same area as previously shown in Figure 3. It is assumed that during the initial clockwise trip around the area, the cable was fully dispensed at point X. The machine has returned to the start point at the socket 32. In this situation, the control system will not yet have received a signal from the second detector 33. Thus, the control system is arranged to recognise that a subsequent signal from the first detector 22 indicates that a further cleaning operation needs to be effected in the remaining part of the room. Figure 6 shows the machine on its anti-clockwise journey around the boundary. The machine begins 'spiking' as soon as it returns to the home position indicated by the marker 21. The machine will continue in this manner until either the cable 9 is again fully dispensed or the navigation system detects that point X has been reached or passed.

Figure 7 shows an alternative scheme in which the machine, once it has returned to the start point at the socket 32, begins to travel around the boundary in the anti-clockwise direction. However, instead of immediately beginning to spike into the area, it simply travels around the boundary, dispensing cable, until the navigation system detects that point X has been reached or passed. The reason for this difference is because it may be easier for the machine to detect when it reaches point X if the machine travels there directly as there will then be fewer accumulated odometry errors.

Although the invention has been described with reference to a machine arranged to move according to a spike pattern, the control system may be adapted to employ other types of manoeuvre. For example, in the case of a machine arranged to move in a spiral pattern, the control system may be arranged to cause the machine to take an inward step on detecting that a circuit of the room has been completed.

The second detector 33 may be omitted. In this embodiment, the control system is arranged to recognise that a circuit of the room has been completed when the detector 22 detects the marker on a second occasion. This will occur when the machine has passed the marker 21 and is just about to embark on a second circuit of the room. Thus, it is preferred to provide the second detector to prevent the machine from treating an area that has already been treated. Of course, a plurality of detectors and markers may be employed.

As a further alternative, the marker may be located on the main body, with the detector being spaced from that main body by, for example, being located on the power cable. A second marker may be placed on the main body, opposite the first marker, for detecting when the machine has completed a circuit. This alternative arrangement has the advantage of requiring just one sensor. However, means for conveying signals from the detector to the control system must be provided, as they are remote from each other. Therefore, in general, the arrangement of having the or each detector on the main body, and the marker spaced from that main body, is preferred.

The marker and detector need not operate by generating and detecting a magnetic field. The marker may be a source of infrared radiation, with the detector being an infrared detector. The marker may emit ultrasonic vibrations, in which case the detector is arranged to detect ultrasound. Alternatively, the marker may simply present a physical obstacle, with the detector being of the bump sensor type having a resilient part arranged to send a signal to the control system when urged against the marker as the cable is rewound.

## Claims

1. An autonomous machine (1) comprising a main body (2) a power cable (9) for providing power to the main body, a marker (21) and a control system arranged to cause the main body to traverse a working area according to a predetermined sequence, the control system including a detect (22,33) arranged to detect the marker, one of the detector and the marker being located on the main body, the other of the detector and the marker being locatable at a plurality of positions on the cable, and the control system being arranged to recognise a predetermined location in the area, or to effect a predetermined part of the sequence, on detecting the marker.

2. An autonomous machine as claimed in claim 1, wherein the predetermined part of the sequence comprises the start of the sequence.

3. An autonomous machine as claimed in claim 1 or 2, wherein the predetermined part of the sequence comprises causing the main body to return to a starting point in the working area.

4. An autonomous machine as claimed in claim 1, 2 or 3, wherein the predetermined part of the sequence comprises the end of the sequence.

5. An autonomous machine as claimed in any preceding claim, wherein the other of the detector and the marker is slidably movable along the cable.

6. An autonomous machine as claimed in any preceding claim, wherein the one of the detector and the marker is located on the main body adjacent a region where the power cable attaches to, or enters, the main body.

7. An autonomous machine as claimed in any preceding claim, wherein the marker is magnetic and the detector is arranged to detect a magnetic field.

8. An autonomous machine as claimed in claim 7, wherein the detector includes a Hall effect sensor.

9. An autonomous machine as claimed in claim 7, wherein the detector includes a reed switch.

10. An autonomous machine as claimed in any preceding claim, further comprising a second detector or marker located on the main body.

11. An autonomous machine as claimed in any preceding claim, wherein the main body is powered by an external power supply and the main body stores a length of power cable that is connectable to the external supply.

12. An autonomous machine as claimed in claim 11, wherein the main body is arranged to dispense and rewind the power cable.

13. A method of operating an autonomous machine (1) comprising a main body (2), a power cable (9) for providing power to the main body, a marker (21) and a control system including a detector (22,33) arranged to detect the marker, the control system being arranged to cause the main body to traverse a working area according to a predetermined sequence, the method comprising the user dispensing a portion of the length of cable and locating the marker or detector at a convenient position on the portion of the cable, and the control system recognising a predetermined location in the working area, or effecting a predetermined part of the sequence, on detecting the marker.

14. A method as claimed in claim 13, further comprising the initial step of a user connecting the power cable to an external power supply.

15. A method as claimed in claim 13 or 14, wherein the user locates the marker or detector at the convenient position by sliding it along the portion of cable.

16. A method as claimed in any one of claims 13 to 15, wherein the predetermined part of the sequence comprises the start of the sequence.

17. A method as claimed in any one of claims 13 to 16, wherein the predetermined part of the sequence comprises controlling the body to return to a starting point

18. A method as claimed in any one of claims 13 to 17, wherein the predetermined part of the sequence comprises the end of the sequence

19. An autonomous machine, as claimed in any one of claims 1 to 12, in the form of a vacuum cleaner.

## Patentansprüche

1. Autonome Maschine (1), die aufweist: einen Hauptkörper (2); ein Stromkabel (9) für das Liefern von Strom zum Hauptkörper; eine Markierung (21); und ein Steuersystem, das angeordnet ist, um den Hauptkörper zu veranlassen, einen Arbeitsbereich entsprechend einem vorgegebenen Arbeitsablauf zu durchqueren, wobei das Steuersystem einen Detektor (22, 33) einschließt, der angeordnet ist, um die Markierung zu erkennen, wobei eines von Detektor und Markierung am Hauptkörper angeordnet ist, wobei das andere von Detektor und Markierung an einer Vielzahl von Positionen am Kabel angeordnet werden kann, und wobei das Steuersystem angeordnet ist, um eine vorgegebene Stelle im Bereich zu erkennen, oder um einen vorgegebenen Abschnitt des Arbeitsablaufes beim Erkennen der Markierung zu bewirken.

2. Autonome Maschine nach Anspruch 1, bei der der vorgegebene Abschnitt des Arbeitsablaufes den Start des Arbeitsablaufes aufweist.

3. Autonome Maschine nach Anspruch 1 oder 2, bei der der vorgegebene Abschnitt des Arbeitsablaufes das Veranlassen des Hauptkörpers zur Rückkehr zu einem Ausgangspunkt im Arbeitsbereich aufweist.

4. Autonome Maschine nach Anspruch 1, 2 oder 3, bei der der vorgegebene Abschnitt des Arbeitsablaufes das Ende des Arbeitsablaufes aufweist.

5. Autonome Maschine nach einem der vorhergehenden Ansprüche, bei der das andere von Detektor und Markierung längs des Kabels verschiebbar beweglich ist.

6. Autonome Maschine nach einem der vorhergehenden Ansprüche, bei der das eine von Detektor und Markierung am Hauptkörper benachbart einem Bereich angeordnet ist, wo das Stromkabel am Hauptkörper befestigt ist oder in diesen gelangt.

7. Autonome Maschine nach einem der vorhergehenden Ansprüche, bei der die Markierung magnetisch ist und der Detektor angeordnet ist, um ein Magnetfeld zu erkennen.

8. Autonome Maschine nach Anspruch 7, bei der der Detektor einen Hallsensor einschließt.

9. Autonome Maschine nach Anspruch 7, bei der der Detektor einen Reed-Schalter einschließt.

10. Autonome Maschine nach einem der vorhergehenden Ansprüche, die außerdem einen zweiten Detektor oder Markierung aufweist, am Hauptkörper angeordnet.

11. Autonome Maschine nach einem der vorhergehenden Ansprüche, bei der der Hauptkörper durch eine externe Stromversorgung mit Strom versorgt wird und der Hauptkörper eine Länge des Stromkabels aufbewahrt, das mit der externen Versorgung verbunden werden kann.

12. Autonome Maschine nach Anspruch 11, bei der der Hauptkörper angeordnet ist, um das Stromkabel auszugeben und wieder aufzuwickeln.

13. Verfahren zum Betätigen einer autonomen Maschine (1), die einen Hauptkörper (2), ein Stromkabel (9) für das Liefern von Strom zum Hauptkörper, eine Markierung (21) und ein Steuersystem einschließlich eines Detektors (22, 33) aufweist, der angeordnet ist, um die Markierung zu erkennen, wobei das Steuersystem so angeordnet ist, dass veranlasst, dass der Hauptkörper einen Arbeitsbereich entsprechend einem vorgegebenen Arbeitsablauf durchquert, wobei das Verfahren die folgenden Schritte aufweist: Ausgeben eines Abschnittes der Länge des Kabels durch den Benutzer; und Anordnen der Markierung oder des Detektors in einer geeigneten Position auf dem Abschnitt des Kabels, wobei das Steuersystem eine vorgegebene Stelle im Arbeitsbereich erkennt oder einen vorgegebenen Abschnitt des Arbeitsablaufes beim Erkennen der Markierung bewirkt.

14. Verfahren nach Anspruch 13, das außerdem den anfänglichen Schritt des Anschließens des Stromkabels an eine externe Stromversorgung durch den Benutzer aufweist.

15. Verfahren nach Anspruch 13 oder 14, bei dem der Benutzer die Markierung oder den Detektor in einer geeigneten Position anordnet, indem sie ihn längs des Abschnittes des Kabels verschiebt.

16. Verfahren nach einem der Ansprüche 13 bis 15, bei dem der vorgegebene Abschnitt des Arbeitsablaufes den Start des Arbeitsablaufes aufweist.

17. Verfahren nach einem der Ansprüche 13 bis 16, bei dem der vorgegebene Abschnitt des Arbeitsablaufes das Steuern des Körpers aufweist, um zu einem Ausgangspunkt zurückzukehren.

18. Verfahren nach einem der Ansprüche 13 bis 17, bei dem der vorgegebene Abschnitt des Arbeitsablaufes das Ende des Arbeitsablaufes aufweist.

19. Autonome Maschine nach einem der Ansprüche 1 bis 12 in der Form eines Staubsaugers.

## Revendications

1. Machine autonome (1), comprenant un corps principal (2), un câble d'alimentation (9) pour fournir de l'énergie au corps principal, un marqueur (21) et un système de commande, destiné à entraîner le corps principal à traverser une zone de travail en fonction d'une séquence prédéterminée, le système de commande englobant un détecteur (22, 33) destiné à détecter le marqueur, l'un d'entre le détecteur et le marqueur, étant agencé sur le corps principal, l'autre d'entre le détecteur et le marqueur, pouvant être agencé au niveau de plusieurs positions sur le câble, et le système de commande étant agencé de sorte à reconnaître un emplacement prédéterminé dans la zone, ou à exécuter une partie prédéterminée de la séquence, lors de la détection du marqueur.

2. Machine autonome selon la revendication 1, dans laquelle la partie prédéterminée de la séquence comprend le démarrage de la séquence.

3. Machine autonome selon les revendications 1 ou 2, dans laquelle la partie prédéterminée de la séquence comprend l'entraînement du corps principal à retourner vers un point de départ dans la zone de travail.

4. Machine autonome selon les revendications 1, 2 ou 3, dans laquelle la partie prédéterminée de la séquence comprend la fin de la séquence.

5. Machine autonome selon l'une quelconque des revendications précédentes, dans laquelle l'autre d'entre le détecteur et le marqueur, peut être déplacé de manière coulissante le long du câble.

6. Machine autonome selon l'une quelconque des revendications précédentes, dans laquelle l'un d'entre le détecteur et le marqueur, est agencé sur le corps principal près d'une région dans laquelle le câble d'alimentation est fixé sur le corps principal ou rentre dans celui-ci.

7. Machine autonome selon l'une quelconque des revendications précédentes, dans laquelle le marqueur est magnétique, le détecteur étant destiné à détecter un champ magnétique.

8. Machine autonome selon la revendication 7, dans laquelle le détecteur englobe un capteur à effet Hall.

9. Machine autonome selon la revendication 7, dans laquelle le détecteur englobe un commutateur à lames.

10. Machine autonome selon l'une quelconque des revendications précédentes, comprenant en outre un deuxième détecteur ou marqueur agencé sur le corps principal.

11. Machine autonome selon l'une quelconque des revendications précédentes, dans laquelle le corps principal est actionné par un bloc d'alimentation en énergie externe, le corps principal stockant une longueur du câble d'alimentation pouvant être connecté au bloc d'alimentation externe.

12. Machine autonome selon la revendication 11, dans laquelle le corps principal est destiné à dérouler et à réenrouler le câble d'alimentation.

13. Procédé d'actionnement d'une machine autonome (1), comprenant un corps principal (2), un câble d'alimentation (9) pour fournir de l'énergie au corps principal, un marqueur (21) et un système de commande englobant un détecteur (22, 33) destiné à détecter le marqueur, le système de commande étant destiné à entraîner le corps principal à traverser une zone de travail en fonction d'une séquence prédéterminée, le procédé comprenant les étapes de déroulement d'une partie de la longueur du câble par l'utilisateur et de positionnement du marqueur ou du détecteur au niveau d'une position appropriée sur la partie du câble, le système de commande reconnaissant un emplacement prédéterminé dans la zone de travail ou exécutant une partie prédéterminée de la séquence lors de la détection du marqueur.

14. Procédé selon la revendication 13, comprenant en outre l'étape initiale de connexion du câble d'alimentation à un bloc d'alimentation en énergie externe par un utilisateur.

15. Procédé selon les revendications 13 ou 14, dans lequel l'utilisateur positionne le marqueur ou le détecteur au niveau d'une position appropriée en le faisant glisser le long d'une partie du câble.

16. Procédé selon l'une quelconque des revendications 13 à 15, dans lequel la partie prédéterminée de la séquence comprend le démarrage de la séquence.

17. Procédé selon l'une quelconque des revendications 13 à 16, dans lequel la partie prédéterminée de la séquence comprend l'entraînement du corps à retourner vers un point de départ.

18. Procédé selon l'une quelconque des revendications 13 à 17, dans lequel la partie prédéterminée de la séquence comprend la fin de la séquence.

19. Machine autonome selon l'une quelconque des revendications 1 à 12, sous forme d'un aspirateur.
